# EUROPEAN PATENT APPLICATION

(11) **EP 1 316 605 A2**
(43) Date of publication of application: **04.06.2003**
(21) Application number: 02257862.9
(22) Date of filing: 14.11.2002
(51) Int. Cl.: C12N 9/16, C12N 15/11, C12N 15/66

(54) **Method for producing DNA**

(30) Priority: 16.11.2001 JP 2001351927
(71) Applicant: NISSHINBO INDUSTRIES, INC., Chuo-ku, Tokyo (JP)
(72) Inventor: Aotsuka, Satoshi, c/o Nisshinbo Industries, Inc., Chiba-shi, Chiba (JP)
(74) Representative: Tombling, Adrian George

(57) **Abstract**

A vector comprising one recognition sequence of a first restriction enzyme of which digestion site exists at a particular position with respect to the recognition sequence and does not exist within the recognition sequence and one recognition sequence of a second restriction enzyme of which digestion site is specific, wherein a fragment obtained by digestion of the vector with the first restriction enzyme can be digested with the second restriction enzyme and a distance between the digestion site of the first restriction enzyme and the digestion site of the second restriction enzyme is 30 nucleotides or shorter, and a method for producing DNA having a target sequence by using the vector.

Sequence Listing

<110> Nisshinbo Industries, Inc.

<120> Method for producing DNA

<130>

<150> JP 2001-351927
<151> 2001-11-16

<160> 22

<210> 1
<211> 12
<212> DNA
<213> Artificial Sequence

<220>
<223> synthetic DNA

<400> 1
cagctcgaat tc                                                          12

<210> 2
<211> 11
<212> DNA
<213> Artificial Sequence

<220>
<223> synthetic DNA

<400> 2
cagctctcga g                                                           11

<210> 3
<211> 12
<212> DNA
<213> Artificial Sequence

<220>
<223> synthetic DNA

<400> 3
gaggagccgc gg                                                          12

<210> 4
<211> 13
<212> DNA
<213> Artificial Sequence

<220>
<223> synthetic DNA

<220>
<221> misc_feature
<222> 6, 7
<223> n=a, g, t or c

<400> 4
ggatgnncag ctc                                                    13

<210> 5
<211> 13
<212> DNA
<213> Artificial Sequence

<220>
<223> synthetic DNA

<220>
<221> misc_feature
<222> 7
<223> n=a, g, t or c

<400> 5
ctggagncgt ctc                                                    13

<210> 6
<211> 12
<212> DNA
<213> Artificial Sequence

<220>
<223> synthetic DNA

<400> 6
gaattcggtc tc                                                     12

<210> 7
<211> 10
<212> DNA

<213> Artificial Sequence

<220>
<223> synthetic DNA

<400> 7
ccatggtctc                                                            10

<210> 8
<211> 25
<212> DNA
<213> Artificial Sequence

<220>
<223> synthetic DNA

<220>
<221> misc_feature
<222> 7, 8, 9, 10, 11, 12, 19
<223> n=a, g, t or c

<400> 8
cagctcnnnn nngaattcng agctg                                           25

<210> 9
<211> 18
<212> DNA
<213> Artificial Sequence

<220>
<223> synthetic DNA

<220>
<221> misc_feature
<222> 7, 8, 9, 10, 11, 12
<223> n=a, g, t or c

<400> 9
cagctcnnnn nngaattc                                                   18

<210> 10
<211> 13
<212> DNA
<213> Artificial Sequence

<220>

<223> synthetic DNA

<220>
<221> misc_feature
<222> 7
<223> n=a, g, t or c

<400> 10
gaattcngag ctg 13

<210> 11
<211> 20
<212> DNA
<213> Artificial Sequence

<220>
<223> synthetic DNA

<220>
<221> misc_feature
<222> 7, 14
<223> n=a, g, t or c

<400> 11
cagctcngaa ttcngagctg 20

<210> 12
<211> 18
<212> DNA
<213> Artificial Sequence

<220>
<223> synthetic DNA

<220>
<221> misc_feature
<222> 7
<223> n=a, g, t or c

<400> 12
cagctcngaa ttcaattc 18

<210> 13
<211> 13
<212> DNA
<213> Artificial Sequence

<220>
<223> synthetic DNA

<220>
<221> misc_feature
<222> 7
<223> n=a, g, t or c

<400> 13
gaattcngag ctg                                                          13

<210> 14
<211> 23
<212> DNA
<213> Artificial Sequence

<220>
<223> synthetic DNA

<220>
<221> misc_feature
<222> 7, 8, 9, 10, 11, 12, 14, 15, 16, 17
<223> n=a, g, t or c

<400> 14
cagctcnnnn nntnnnnccc agt                                               23

<210> 15
<211> 18
<212> DNA
<213> Artificial Sequence

<220>
<223> synthetic DNA

<220>
<221> misc_feature
<222> 7, 8, 9, 10, 11, 12
<223> n=a, g, t or c

<400> 15
cagctcnnnn nntaattc                                                     18

<210> 16
<211> 13

<212> DNA
<213> Artificial Sequence

<220>
<223> synthetic DNA

<220>
<221> misc_feature
<222> 2, 3, 4, 5, 6, 7
<223> n=a, g, t or c

<400> 16
annnnnngag ctg                                                    13

<210> 17
<211> 18
<212> DNA
<213> Artificial Sequence

<220>
<223> synthetic DNA

<220>
<221> misc_feature
<222> 7, 11, 12, 14, 15
<223> n=a, g, t or c

<400> 17
cagctcngac nntnngtc                                               18

<210> 18
<211> 18
<212> DNA
<213> Artificial Sequence

<220>
<223> synthetic DNA

<220>
<221> misc_feature
<222> 7, 11, 12
<223> n=a, g, t or c

<400> 18
cagctcngac nntaattc                                               18

<210> 19
<211> 13
<212> DNA
<213> Artificial Sequence

<220>
<223> synthetic DNA

<220>
<221> misc_feature
<222> 2, 3, 7
<223> n=a, g, t or c

<400> 19
anngtcngag ctg 13

<210> 20
<211> 12
<212> DNA
<213> Artificial Sequence

<220>
<223> synthetic DNA

<400> 20
gtcttcgaat tc 12

<210> 21
<211> 12
<212> DNA
<213> Artificial Sequence

<220>
<223> synthetic DNA

<220>
<221> misc_feature
<222> 12
<223> n=a, g, t or c

<400> 21
   aattcggtct cn                                                     12

<210> 22
<211> 10
<212> DNA

<213> Artificial Sequence

<220>
<223> synthetic DNA

<220>
<221> misc_feature
<222> 10
<223> n=a, g, t or c

<400> 22
catggtctcn                                                                10

## Description

### BACKGROUND OF THE INVENTION

The present invention relates to a method for producing DNA having an arbitrary sequence. More specifically, the present invention relates to a method for producing DNA by ligation.

As a method for producing DNA, there are known a method based on PCR, a method based on chemical synthesis reactions using an automatic synthesizer and so forth.

However, the method based on PCR suffers from a limitation that DNA having a target nucleotide sequence to be used as a template needs to exist beforehand. On the other hand, the method based on chemical synthesis reactions does not suffer from such a limitation. However, since a length of DNA that can be practically produced by the method has an upper limit, DNA having a length longer than the limit must be produced by ligation.

As a method for producing DNA by ligation, there is known a method of synthesizing, through chemical synthesis reactions, pairs of single-stranded DNA's having such sequences that DNA's of each pair generates a unique cohesive end when they are annealed and ligating the double-stranded DNA's obtained by annealing DNA's of each pair by using a ligase (Advances in Biochemical Engineering/Biotechnology, Vol. 37, 73-127 (1988)). However, in this method, since double-stranded DNA's having a unique cohesive end must be generated, it is difficult to confirm the nucleotide sequence of the each product in the course of the process. In addition, since impurities that can exist in DNA synthesized by the chemical synthesis reactions or erroneous annealing are all reflected in the final product, the probability of obtaining a final product having the target sequence becomes low, and a problem arises in view of efficiency.

As a method free from the aforementioned problems, there is known a method of preparing relatively short fragments with an end having the same shape as that of an end formed by digestion with a restriction enzyme by the aforementioned ligation method, cloning the fragments in an appropriate vector and then successively ligating fragments excised from the vector using a restriction enzyme (Advances in Biochemical Engineering/Biotechnology, Vol. 37, 73-127 (1988)). However, in this method, as the number of fragments to be ligated increases, the number of required recognition sites of the restriction enzymes correspondingly increases. Therefore, the method is not efficient in regard of increased number of steps for temporarily introducing or eliminating recognition sites of the restriction enzymes in the target sequence.

### SUMMARY OF THE INVENTION

An object of the present invention is to provide an efficient method for producing DNA by ligation of DNA fragments.

The inventors of the present invention found that DNA having a target nucleotide sequence could be efficiently produced by using a vector in which restriction enzyme sites are arranged in a specific manner, and thus accomplished the present invention.

That is, the present invention provides the following:
1. A vector comprising one recognition sequence of a first restriction enzyme of which digestion site exists at a particular position with respect to the recognition sequence and does not exist within the recognition sequence and one recognition sequence of a second restriction enzyme of which digestion site is specific, wherein a fragment obtained by digestion of the vector with the first restriction enzyme can be digested with the second restriction enzyme and a distance between the digestion site of the first restriction enzyme and the digestion site of the second restriction enzyme is 30 nucleotides or shorter.
2. A method for producing DNA having a target sequence, comprising steps of:
   (1) preparing a plurality of double-stranded DNA fragments each having one of continuous partial sequences obtained by dividing the target sequence as a part thereof,
   (2) preparing the vector as defined in the aforementioned 1,
   (3) digesting the vector with the first restriction enzyme,
   (4) digesting the fragment obtained by the digestion with the first restriction enzyme with the second restriction enzyme,
   (5) ligating a longer fragment out of the fragments obtained by the digestion with the second restriction enzyme and one of the DNA fragments prepared in the step (1) in a predetermined order, and
   (6) repeating the steps of (3) to (5) by using a clone obtained by the ligation as the vector in the step (3) until all the DNA fragments prepared in the step (1) are ligated, wherein
      adjacent partial sequences overlap by nucleotides in a number of nucleotides in a protruding segment of a cohesive end to be generated at the digestion site of the first restriction enzyme,
      one end of each DNA fragment prepared in the step (1) is formed by adding a sequence to each partial sequence, the added sequence forming an end ligatable to an end formed by digestion with the second restriction enzyme, to which the DNA fragment is ligated, and the added sequence having such a length that digestion occurs at an end of the partial sequence upon digestion with the first restriction enzyme after the ligation,
      the other end of each DNA fragment is ligatable to an end formed by digestion with the first restriction enzyme, to which the DNA fragment is ligated, and
      the first restriction enzyme and the second restriction enzyme are selected so that their recognition sequences do not exist within the ligated partial sequence.
3. The method according to the aforementioned 2, which further comprises, when a sequence identical to the recognition sequence of the first restriction enzyme or the second restriction enzyme exists within the target sequence, a step of preparing the DNA fragments in the step (1) with changing the identical sequence to a different sequence and restoring the changed segment in the sequence of the DNA fragment obtained in the step (6) to the original sequence to produce DNA having the target sequence.
4. A method for producing DNA having a target sequence, comprising steps of:
   (1) preparing a plurality of double-stranded DNA fragments each having one of continuous partial sequences obtained by dividing the target sequence as a part thereof,
   (2) preparing the vector as defined in the aforementioned 1, to which a DNA fragment being one of the DNA fragments prepared in the step (1) and having one of end partial sequences of the target sequence is inserted so that digestion occurs at an end of the partial sequence upon digestion with the first restriction enzyme,
   (3) digesting the vector with the first restriction enzyme,
   (4) digesting the fragment obtained by the digestion with the first restriction enzyme with the second restriction enzyme,
   (5) ligating a longer fragment out of the fragments obtained by the digestion with the second restriction enzyme and one of the DNA fragments prepared in the step (1), and
   (6) repeating the steps of (3) to (5) by using a clone obtained by the ligation as the vector in the step (3) until all the DNA fragments prepared in the step (1) are ligated, wherein
      adjacent partial sequences overlap by nucleotides in a number of nucleotides in a protruding segment of a cohesive end to be generated at the digestion site of the first restriction enzyme,
      one end of each of the DNA fragments that are not the DNA fragment inserted in the step (2) is formed by adding a sequence to each partial sequence, the added sequence forming an end ligatable to an end formed by digestion with the second restriction enzyme, to which the DNA fragment is ligated, and the added sequence having such a length that digestion occurs at an end of the partial sequence upon digestion with the first restriction enzyme after the ligation,
      the other end of each of the DNA fragments that are not the DNA fragment inserted in the step (2) is ligatable to an end formed by digestion with the first restriction enzyme, to which the DNA fragment is ligated, and
      the first restriction enzyme and the second restriction enzyme are selected so that their recognition sequences do not exist within the ligated partial sequences.
5. The method according to the aforementioned 4, which further comprises, when a sequence identical to the recognition sequence of the first restriction enzyme or the second restriction enzyme exists within the target sequence, a step of preparing the DNA fragments in the step (1) with changing the identical sequence to a different sequence and restoring the changed segment in the sequence of the DNA fragment obtained in the step (6) to the original sequence to produce DNA having the target sequence.
6. A method for producing a DNA fragment used in the production method as defined in any one of the aforementioned 2 to 5, in which one end of the DNA fragment is formed by adding a sequence to a partial sequence, said added sequence forming an end ligatable to an end formed by digestion with the second restriction enzyme, to which the DNA fragment is ligated, and the added sequence having such a length that digestion occurs at an end of the partial sequence upon digestion with the first restriction enzyme after the ligation, and the other end is an end ligatable to an end formed by digestion with the first restriction enzyme, to which the DNA fragment is ligated, which method comprises steps of:
   (a) preparing a vector having a recognition sequence of a third restriction enzyme which generates a digestion site having the same shape as that of a digestion site of the first restriction enzyme, and a recognition sequence of a fourth restriction enzyme, in which the recognition sequence of the fourth restriction enzyme exists between the recognition sequence and the digestion site of the third restriction enzyme,
   (b) preparing a fragment comprising a partial sequence of which both ends are ligatable to an end formed by digestion with the fourth restriction enzyme,
   (c) digesting the vector prepared in the step (a) with the fourth restriction enzyme and ligating the fragment obtained by the digestion with the fourth restriction enzyme and the DNA fragment prepared in the step (b),
   (d) analyzing nucleotide sequences of clones obtained by the ligation to select a clone having the target partial sequence, and
   (e) successively digesting the selected clone with the third restriction enzyme and the fourth restriction enzyme,
      wherein
      sequences serving as the recognition sequences of the third restriction enzyme and the fourth restriction enzyme are provided in the vector and the fragment prepared in the steps (a) and (b), respectively, so that a predetermined DNA fragment is excised upon digestion of the clones obtained in the step (c) with the third restriction enzyme.
7. The method according to the aforementioned 6, wherein the recognition sequence of the third restriction enzyme and the recognition sequence of the fourth restriction enzyme are identical,
   the vector prepared in the step (a) has another recognition sequence of the third restriction enzyme, digestion with the third restriction enzyme based on this recognition sequence generates an end having the same shape as that of an end formed by digestion with the fourth restriction enzyme, two recognition sequences of the third restriction enzyme exist on the both sides of the digestion site of the fourth restriction enzyme, and
   digestion is performed only with the third restriction enzyme in the step (e).
8. The method according to the aforementioned 6, wherein the vector prepared in the step (a) has another recognition sequence of the third restriction enzyme, and two recognition sequences of the third restriction enzyme are symmetrically positioned as also for their directions with respect to the recognition sequence of the fourth restriction enzyme, and
   digestion is performed only with the third restriction enzyme in the step (e).
9. The method according to the aforementioned 8, wherein the vector prepared in the step (a) has two recognition sequences of the fourth restriction enzyme, two recognition sequences of the fourth restriction enzyme are positioned in directions inverse to each other, the recognition sequences of the third restriction enzyme are positioned symmetrically as also for their directions on both sides of two recognition sequences of the fourth restriction enzyme, and the fourth restriction enzyme forms a one nucleotide-protruding end at the 3' end.
10. A method for producing a DNA fragment used in the production method as defined in any one of the aforementioned 2 to 5, in which one end of the DNA fragment is formed by adding a sequence to a partial sequence, the added sequence forming an end ligatable to an end formed by digestion with the second restriction enzyme, to which the DNA fragment is ligated, and the added sequence having such a length that digestion occurs at an end of the partial sequence upon digestion with the first restriction enzyme after the ligation, and the other end is an end ligatable to an end formed by digestion with the first restriction enzyme, to which the DNA fragment is ligated, which method comprises steps of:
   (a) preparing a vector having a recognition sequence of the fourth restriction enzyme,
   (b) preparing a fragment containing a partial sequence of which both ends are ligatable to an end formed by digestion with the fourth restriction enzyme, which has a recognition sequence of the third restriction enzyme so that digestion based on the recognition sequence of the third restriction enzyme generating a digested site having the same shape as that of a digested site based on the recognition sequence of the first restriction enzyme generates an end having the same shape as that of one end of the fragment, and has a recognition sequence of a fifth restriction enzyme generating an end having the same shape as an end generated by the third restriction enzyme so that digestion with the fifth restriction enzyme occurs at an end of the partial sequence,
   (c) digesting the vector prepared in the step (a) with the fourth restriction enzyme and ligating the fragment obtained by the digestion with the fourth restriction enzyme and the DNA fragment prepared in the step (b),
   (d) analyzing nucleotide sequences of clones obtained by the ligation to select a clone having the target partial sequence, and
   (e) digesting the selected clone with the fourth restriction enzyme and the fifth restriction enzyme,
      wherein
      a sequence serving as the recognition sequence of the fifth restriction enzyme is provided in the vector and the fragment prepared in the steps (a) and (b), respectively, so that a predetermined DNA fragment is excised upon digestion of the clones obtained in the step (c) with the fifth restriction enzyme.
11. A vector used for the method as defined in the aforementioned 7, which comprises the recognition sequence of the third restriction enzyme, of which digestion site exists at a particular position with respect to the recognition sequence and does not exists within the recognition sequence, and the recognition sequence of the fourth restriction enzyme of which digestion site is specific, and in which the recognition sequence of the fourth restriction enzyme exists between the recognition sequence and the digestion site of the third restriction enzyme, wherein the vector has another recognition sequence of the third restriction enzyme, digestion based on this recognition sequence with the third restriction enzyme generates an end having the same shape as that obtained by digestion with the fourth restriction enzyme, and two recognition sequences of the third restriction enzyme exist on the both sides of the digestion site of the fourth restriction enzyme.
12. A vector used for the method as defined in the aforementioned 8, which comprises the recognition sequence of the third restriction enzyme, of which digestion site exists at a particular position with respect to the recognition sequence and does not exists within the recognition sequence, and the recognition sequence of the fourth restriction enzyme of which digestion site is specific, and in which the recognition sequence of the fourth restriction enzyme exists between the recognition sequence and the digestion site of the third restriction enzyme, wherein the vector comprises another recognition sequence of the third restriction enzyme, and two recognition sequences of the third restriction enzyme are symmetrically positioned as also for their directions with respect to the recognition sequence of the fourth restriction enzyme.
13. The vector according to the aforementioned 12, which comprises two recognition sequences of the fourth restriction enzyme, and in which two recognition sequences of the fourth restriction enzyme are positioned in directions inverse to each other, the recognition sequences of the third restriction enzyme are symmetrically positioned as also for their directions on both sides of two recognition sequences of the fourth restriction enzyme, and the fourth restriction enzyme forms a one nucleotide-protruding end at the 3' end.

According to the present invention, it becomes possible to efficiently produce DNA by ligation of DNA fragments.

### BREIF EXPLANATION OF THE DRAWINGS

Fig. 1 shows examples of combinations of the first restriction enzyme and the second restriction enzyme for the vector of the present invention.

Fig. 2 is an explanatory view showing an example of the production method of the present invention.

Fig. 3 is an explanatory view showing a method for producing an insert fragment.

Fig. 4 is an explanatory view showing another example of the production method of the present invention.

Fig. 5 is an explanatory view showing the first example of the method for producing an insert fragment according to the present invention.

Fig. 6 is an explanatory view showing the second example of the method for producing an insert fragment according to the present invention.

Fig. 7 is an explanatory view showing the third example of the method for producing an insert fragment according to the present invention.

Fig. 8 is an explanatory view showing the fourth example of the method for producing an insert fragment according to the present invention.

Fig. 9 is an explanatory view showing the fifth example of the method for producing an insert fragment according to the present invention.

### DETAILED DESCRIPTION OF THE INVENTION

### <1> Vector of the present invention

The vector of the present invention is a vector comprising one recognition sequence of a first restriction enzyme of which digestion site exists at a particular position with respect to the recognition sequence and does not exist within the recognition sequence and one recognition sequence of a second restriction enzyme, wherein a fragment obtained by digestion of the vector with the first restriction enzyme can be digested with the second restriction enzyme and a distance between the digestion site of the first restriction enzyme and the digestion site of the second restriction enzyme is 30 nucleotides or shorter.

The first restriction enzyme is a restriction enzyme of which digestion site exists at a particular position with respect to its recognition sequence and does not exist within the recognition sequence (hereinafter, also referred to as "distant digestion-type enzyme"). As such a restriction enzyme, Class II restriction enzymes of which digestion site is outside the recognition sequence are known, and specific examples thereof include *Ace*III, *Bse*RI, *Bsa*I, *Fok*I, *Bsm*BI, *Bpm*I, *Mbo*II, *Bbv*I, *Bsg*I, *Bsm*FI, *Eci*I, *Mnl*I *Sfa*NI and so forth.

The second restriction enzyme is not particularly limited so long as its recognition sequence and digestion site are specific. The expression "a digestion site is specific" means that the digestion site exists at a particular position with respect to a recognition site of the enzyme under a specific reaction condition. The digestion site may exist either within or outside the recognition sequence. As such a restriction enzyme, Class II restriction enzymes can be mentioned, and specific examples thereof include *Eco*RI, *Xho*I, *Sac*II, *Nco*I, *Fok*I, *Bsm*BI, *Bmr*I, *Eam*1105I, *Hin*dIII, *Bam*HI, *Kpn*I, *Pst*I, *Xba*I and so forth.

The distance between the digestion site of the first restriction enzyme and the digestion site of the second restriction enzyme means the number of nuclsotides between the end nucleotide position of the protruding strand closer to the digestion site of the second restriction enzyme in the end portion generated by digestion with the first restriction enzyme and the end nucleotide position of the protruding strand closer to the digestion site of the first restriction enzyme in the end portion generated by digestion with the second restriction enzyme.

This distance is 30 nucleotides or shorter, preferably 20 nucleotides or shorter. Ideally, the length should be a minimum length necessary and sufficient for recognition and digestion of DNA with the second restriction enzyme after digestion with the first restriction enzyme. The longer this distance is, the longer the fragment becomes that is removed and wasted in digestion with the first restriction enzyme and the second restriction enzyme. Since it increases the number of nucleotides to be synthesized, production of DNA becomes inefficient.

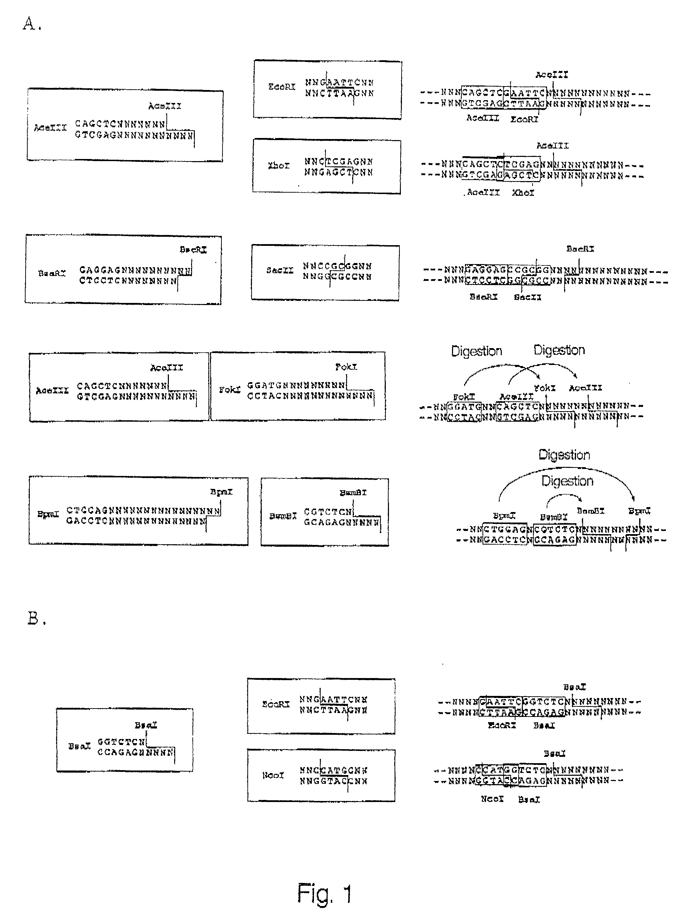

Combination of the first restriction enzyme and the second restriction enzyme is not particularly limited so long as a fragment after digestion with the first restriction enzyme can be digested with the second restriction enzyme, and the distance between the digestion site of the first restriction enzyme and the digestion site of the second restriction enzyme is 30 nucleotides or shorter. Examples of combination of the first restriction enzyme and the second restriction enzyme are shown in Fig. 1. The aforementioned distances for the examples shown in Fig. 1 are shown in Table 1 below. The examples shown in Fig. 1, A are of a type in which the recognition sequence of the second restriction enzyme exits between the recognition sequence and the digestion site of the first restriction enzyme. The examples shown in Fig. 1, B are of a type in which the recognition sequence of the first restriction enzyme exists between the digestion site of the first restriction enzyme and the digestion site of the second restriction enzyme. In this type, the recognition sequence of the first restriction enzyme is eliminated by digestion with the second restriction enzyme after digestion with the first restriction enzyme. The nucleotide sequences of segments containing the recognition sequences in these examples are shown in Sequence Listing (SEQ ID NOS: 1-7).

As shown in the examples shown in Fig. 1, the second restriction enzyme may be either a distant digestion-type enzyme or not. Further, the order of the recognition sequence of the first restriction enzyme and the recognition sequence of the second restriction enzyme is arbitrary, and these sequences may overlap with each other.

The vector of the present invention can be used in the production method of the present invention. Continuous partial sequences obtained by dividing a target sequence are successively inserted between the digestion site of the first restriction enzyme and the digestion site of the second restriction enzyme.

The kinds of the vector are not particularly limited, and the vector may be derived from a plasmid, phage, virus, artificial yeast chromosome or the like. Although the size of the vector is not particularly limited either, a smaller size of the segment other than an inserted sequence is preferred in view of efficient replication.

The vector of the present invention can be produced according to usual gene recombination techniques.

### <2> Production method of the present invention

The production method of the present invention is a method for producing DNA having a target sequence, which comprises the following steps (1) to (6):
(1) preparing a plurality of double-stranded DNA fragments each having one of continuous partial sequences obtained by dividing the target sequence as a part thereof,
(2) preparing the vector according to the present invention,
(3) digesting the vector with the first restriction enzyme,
(4) digesting the fragment obtained by the digestion with the first restriction enzyme with the second restriction enzyme,
(5) ligating a longer fragment out of the fragments obtained by the digestion with the second restriction enzyme and one of the DNA fragments prepared in the step (1) in a predetermined order, and
(6) repeating the steps of (3) to (5) by using a clone obtained by the ligation as the vector in the step (3) until all the DNA fragments prepared in the step (1) are ligated.

In the production method of the present invention,
adjacent partial sequences overlap by nucleotides in a number of nucleotides in a protruding segment of a cohesive end to be generated at the site digested with the first restriction enzyme,
one end of each DNA fragment prepared in the step (1) is formed by adding a sequence to each partial sequence, the added sequence forming an end ligatable to an end formed by digestion with the second restriction enzyme, to which the DNA fragment is ligated, and the added sequence having such a length that digestion occurs at an end of the partial sequence upon digestion with the first restriction enzyme after the ligation,
the other end of each DNA fragment is ligatable to an end formed by digestion with the first restriction enzyme, to which the DNA fragment is ligated, and
the first restriction enzyme and the second restriction enzyme are selected so that their recognition sequences do not exist within the ligated partial sequences.

The first restriction enzyme and the second restriction enzyme are as described in the above explanation of the vector of the present invention.

In the step (1), a plurality of double-stranded DNA fragments each having one of continuous partial sequences obtained by dividing the target sequence as a part thereof are prepared.

The target sequence is not particularly limited. The length of the partial sequence may be a length that can be practically obtained by synthesis based on chemical synthesis reactions or a method using chemical synthesis reactions and PCR in combination. In the case of synthesis based on chemical synthesis reactions, the length is usually 60 to 120 nuclectides, preferably 80 to 100 nucleotides. In the case of the method using synthesis by chemical synthesis reactions and PCR in combination, the length is usually 100 to 220 nucleotides, preferably 140 to 180 nucleotides. The lengths of all these partial sequences do not need to be the same. Adjacent partial sequences overlap by nucleotides in a number of nucleotides in a protruding segment of a cohesive end to be generated at the site digested with the first restriction enzyme. For example, when the first restriction enzyme is *Ace*III, adjacent partial sequences overlap by 4 nucleotides.

One end of a DNA fragment containing each partial sequence is formed by adding a sequence forming an end ligatable to an end formed by digestion with the second restriction enzyme, to which the DNA fragment is ligated, to each partial sequence. Further, the other end is an end ligatable to an end formed by digestion with the first restriction enzyme, to which the DNA fragment is ligated. Consequently, the DNA fragment can be inserted into the vector digested successively with the first restriction enzyme and the second restriction enzyme in a predetermined direction. The expression that the end of the DNA fragment conforms to an end of a partial sequence means that an end of a protruding strand at an end of the DNA fragment conforms to an end of partial sequence.

The sequence added to the end of the DNA fragment has such a length that digestion occurs at an end of the partial sequence upon digestion with the first restriction enzyme after the ligation. Digestion at an end of the partial sequence means that an end of a protruding strand at a digestion end on the partial sequence side becomes an end of the partial sequence. As a result, no irrelevant sequence exists between the partial sequences at an end formed by digestion with the first restriction enzyme.

Further, the adjacent partial sequences overlap by nucleotides in a number of nucleotides in a protruding segment to be generated at an end of the digestion site of the first restriction enzyme. Therefore, DNA fragments that are not the DNA fragment having the partial sequence first inserted into the vector are made to have ends ligatable to an end formed by digestion with the first restriction enzyme by making ends of DNA fragments conform with ends of the partial sequences and making one strand protrudent by a predetermined number of nucleotides.

Following the above procedure, a DNA fragment containing a subsequent partial sequence can be ligated to an end formed by digestion with the first restriction enzyme without inserting irrelevant sequence between the partial sequences.

Specific examples of the sequences that are added to ends of DNA fragments shown in Fig. 1 are listed in Table 1. In the table, N represents an arbitrary nucleotide.

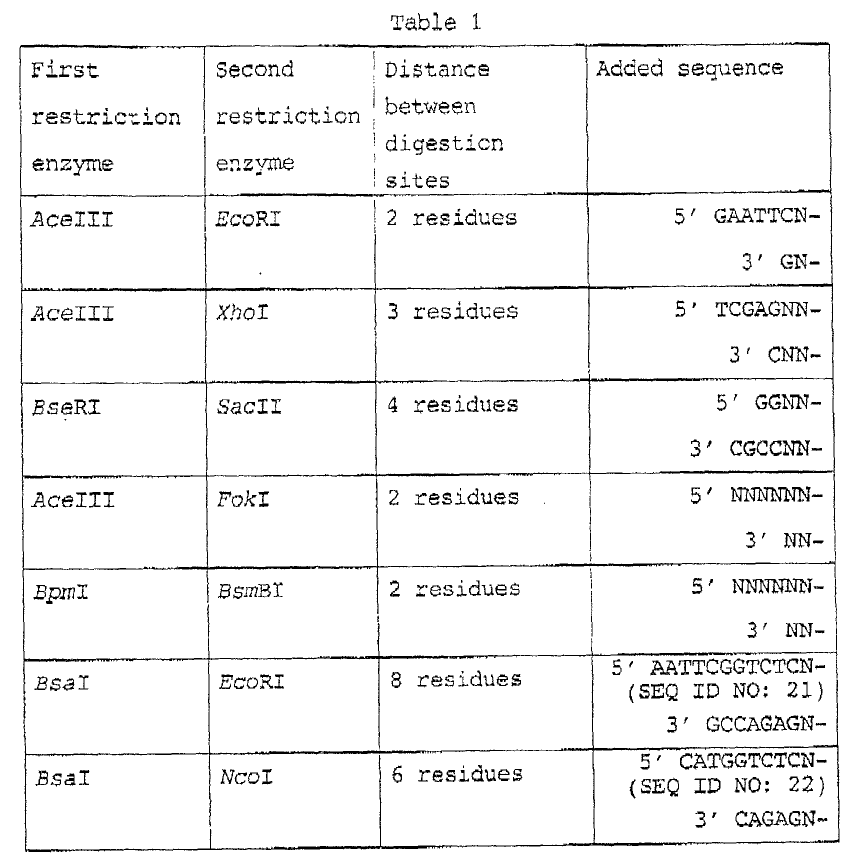

The DNA fragment described above (hereinafter, also referred to as "insert fragment") can be obtained by a usual method such as a method of synthesizing two oligomers through chemical synthesis reactions so that they should form a target fragment upon annealing and then annealing these oligomers and a method of synthesizing two oligomers designed so that a recognition sequence of a restriction enzyme exists on each 5' end side and that their 3' end sides anneal to each other, through chemical synthesis reactions, annealing these oligomers, then performing extension reaction by using a DNA polymersse and digesting the extended fragment with a restriction enzyme. Further, the insert fragments can also be obtained by the insert fragment-producing method of the present invention explained below.

The first restriction enzyme and the second restriction enzyme are usually selected so that their recognition sequences do not exist within the target sequence. However, even when their recognition sequences exist within the target sequence, the production method of the present invention can be applied. In this case, in the step (1), DNA fragments are prepared with changing sequences identical to the recognition sequences of the first restriction enzyme and the second restriction enzyme in the target sequence to different sequences (as a result, the recognition sequences of the first restriction enzyme and the second restriction enzyme do not exist within ligated partial sequences), and further performing a step of restoring the changed segment in the sequence of the DNA fragment obtained in the step (6) to the original sequence to produce DNA having the target sequence. Modification of the sequence for restoring the segment to the original sequence can be performed by a method for introducing a desired mutation such as site-directed mutagenesis.

In the step (2), the aforementioned vector of the present invention is prepared. The vector of the present invention can be produced according to usual gene recombination techniques.

In the step (3), the vector is digested with the first restriction enzyme. The digestion can be performed under conditions suitable for the restriction enzyme used.

In the step (4), the fragment obtained by digestion with the first restriction enzyme is digested with the second restriction enzyme. The digestion, can be performed under conditions suitable for the restriction enzyme used.

In the step (5), a longer fragment out of the fragments obtained by the digestion with the second restriction enzyme and one of the DNA fragments prepared in the step (1) are ligated in a predetermined order. The predetermined order referred to herein means an order of from one end segment to the other end segment of the target sequence. Purification and ligation of the fragments can be performed in a conventional manner.

In the step (6), the steps (3) to (5) are repeated by using the clone obtained by the ligation as the vector used in the step (3) until all the DNA fragments prepared in the step (1) are ligated. Since the clone obtained by the ligation in the step (5) satisfies the requirements of the vector of the present invention, this clone can be used as the vector of the present invention in the step (3).

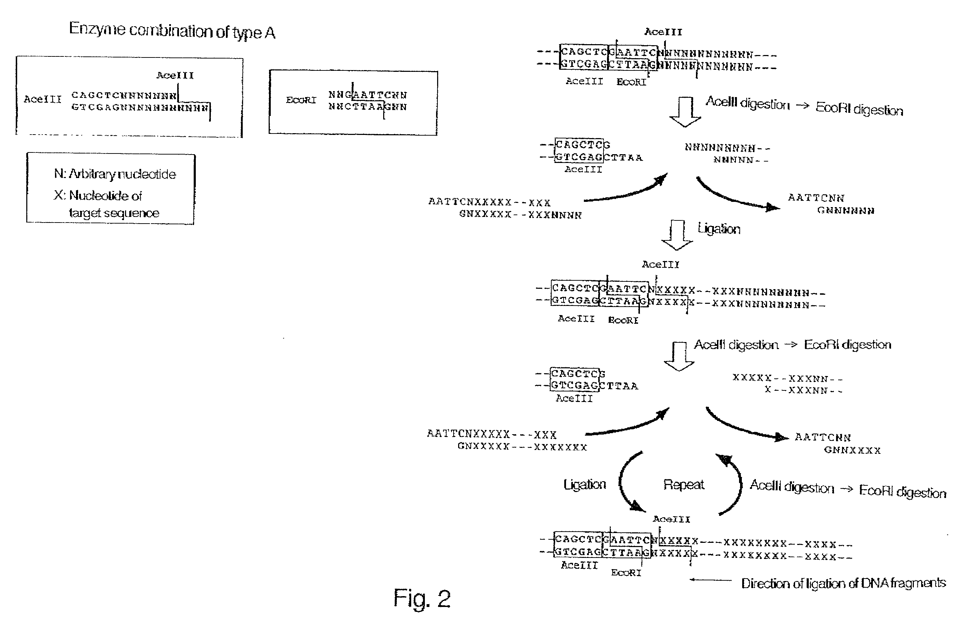

Hereafter, there will be explained a case where *Ace*III and *Eco*RI are selected as the first restriction enzyme and the second restriction enzyme, respectively (those of a type in which the recognition sequence of the second restriction enzyme exists between the recognition sequence and the digestion site of the first restriction enzyme) as an example with reference to Fig. 2. In Fig. 2, nucleotides in the partial sequences obtained by dividing the target sequence are represented by X, and arbitrary nucleotides are represented by N.

First, a DNA fragment having a partial sequence (insert fragment) is prepared. Since the number of nucleotides in the protruding end produced at the digestion site of *Ace*III is 4, adjacent partial sequences overlap by 4 nucleotides. One end of the insert fragment is obtained by adding a sequence forming an end ligatable to an end formed by digestion with *Eco*RI to a partial sequence. The added sequence has such a length that digestion occurs at an end of the partial sequence upon digestion with *Ace*III after ligation. The other end of the insert fragment is an end ligatable to an end formed by digestion with *Ace*III. That is, an end of the insert fragment first inserted has a shape ligatable to an end formed by digestion with *Ace*III in the first vector. When the vector to which the insert fragment is inserted is digested with *Ace*III, an end formed by digestion with *Ace*III is formed at the end of the partial sequence. Therefore, an end of the insert fragment subsequently inserted has such a shape that a segment overlapping with the partial sequence of the previously inserted insert fragment serves as a protruding strand.

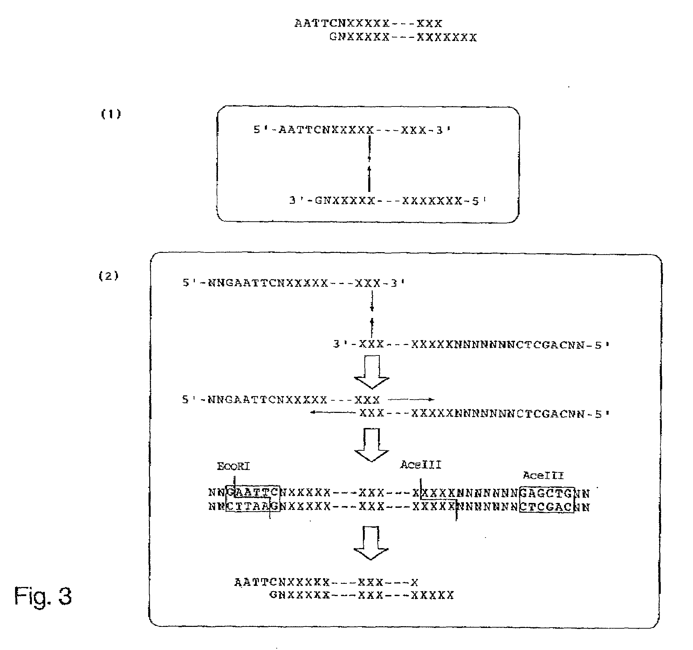

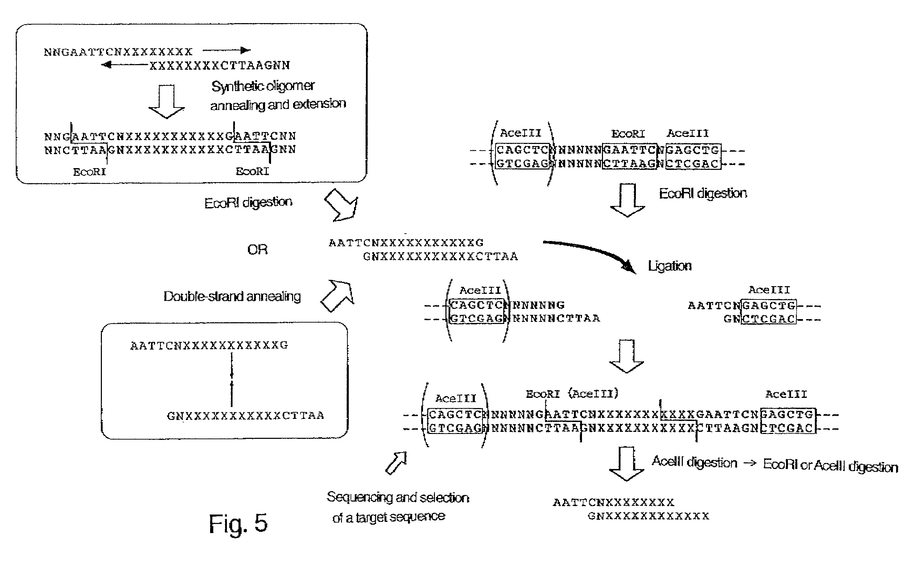

The insert fragment in this example has a sequence shown at the top of Fig. 3. Such an insert fragment can be obtained by a method of synthesizing such two oligomers through chemical synthesis reactions so that they form a target fragment upon annealing and then annealing these oligomers, as outlined in Fig. 3, (1), a method of synthesizing, through chemical synthesis reactions, two oligomers designed so that recognition sequences of *Eco*RI and *Ace*III exist on 5' end sides, respectively, and that their 3' end sides anneal to each other, annealing these oligomers, then performing extension reaction by using a DNA polymerase and digesting the extended fragment with *Eco*RI and *Ace*III, as outlined in Fig. 3, (2), or the like. Further, the insert fragment can also be obtained by the insert fragment-producing method of the present invention explained below (see Figs. 5 to 8).

When the target sequence has the recognition sites of *Ace*III and *Eco*RI, other restriction enzymes are selected. Alternatively, when the partial sequences are designed, the recognition sequences are changed so that the ligated partial sequences do not include the recognition sites of *Ace*III and *Eco*RI.

Further, a vector in which the recognition site of *Eco*RI is positioned between the recognition sequence and the digestion site of *Ace*III is prepared.

After the first vector and the DNA fragments are prepared, the vector is successively digested with *Ace*III and *Eco*RI. This digestion provides two fragments. A longer fragment out of these fragments and one of the DNA fragments (one containing a partial sequence at an end) are ligated to obtain a vector.

Since the vector obtained by the ligation is a vector in which the recognition site of *Eco*RI is positioned between the recognition sequence and the digestion site of *Ace*III, this vector is used in the same manner as the first vector, i.e., it is successively digested with *Ace*III and *Eco*RI as described above and ligated to a subsequent insert fragment (one having a partial sequence adjacent to the partial sequence immediately precedently inserted) to obtain a vector. Until all the prepared insert fragments are ligated, successive digestion with *Ace*III and *Eco*RI and ligation are repeated.

When a sequence is changed in designing partial sequences, the sequence is restored by a method such as site-directed mutagenesis thereafter.

Following the above procedure, DNA having the target sequence is produced.

When a vector of a type in which the recognition sequence of the first restriction enzyme exists between the digestion site of the first restriction enzyme and the digestion site of the second restriction enzyme (see Fig. 1B) is used, one end of each DNA fragment prepared in the step (1) is formed by adding a sequence to a partial sequence, the added sequence forming an end ligatable to an end formed by digestion with the second restriction enzyme, to which the DNA fragment is ligated, and the added sequence having such a length that digestion occurs at an end of the partial sequence upon digestion with the first restriction enzyme after ligation, and the insert fragment contains the recognition sequence of the first restriction enzyme in order to satisfy the requirement that the other end of each DNA fragment has an end ligatable to an end formed by digestion with the first restriction enzyme, to which the DNA fragment is ligated.

In this case, since the insert fragment contains the recognition sequence of the first restriction enzyme, a vector to which a DNA fragment containing the first partial sequence is inserted does not need to have the digestion site of the first restriction enzyme. That is, when a vector of this embodiment is used, the recognition sequence of the first restriction enzyme does not need to be incorporated into the vector beforehand. Furthermore, it is sufficient that the DNA fragment first inserted should be digested at an end of the partial sequence upon digestion with the first restriction enzyme after insertion. For example, it may be a fragment having an end that can be inserted into the digestion site of the second restriction enzyme.

Therefore, in the production method of the present invention using the vector of this embodiment, the aforementioned step (2) can be replaced with a step (2) of preparing the vector of the present invention to which a DNA fragment being one of DNA fragments prepared in the step (1) and having one of the end partial sequences of the target sequence is inserted so that digestion occurs at an end of the partial sequence upon digestion with the first restriction enzyme, wherein one end of each of DNA fragments that are not the DNA fragment inserted in the step (2) is formed by adding a sequence to each partial sequence, the added sequence forming an end ligatabe to an end formed by digestion with the second restriction enzyme, to which the DNA fragment is ligated, and the added sequence having such a length that digestion occurs at an end of the partial sequence upon digestion with the first restriction enzyme after ligation, and the other end of each of DNA fragments that are not the DNA fragment inserted in the step (2) is an end ligatable to an end formed by digestion with the first restriction enzyme, to which the DNA fragment is ligated.

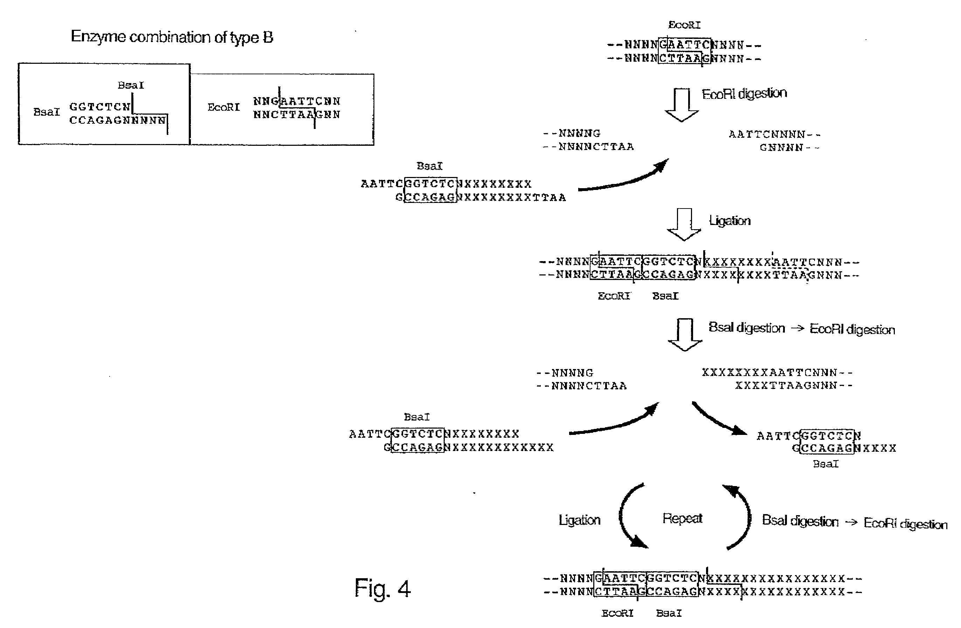

Hereafter, the production method according to this embodiment will be explained with reference to Fig. 4 by using, as an example, a case where *Bsa*I and *Eco*RI are selected as the first restriction enzyme and the second restriction enzyme, respectively (the type in which the recognition sequence of the first restriction enzyme exists between the digestion site of the first restriction enzyme and the digestion site of the second restriction enzyme). In Fig. 4, nucleotides of partial sequences obtained by dividing the target sequence are represented by X, and arbitrary nucleotides are represented by N.

First, a DNA fragment (insert fragment) containing a partial sequence is prepared. Since the number of nucleotides in the protruding end generated at the digestion site of *Bsa*I is 4, adjacent partial sequences overlap by 4 nucleotides. The insert fragment first inserted contains the recognition sequence of *Bsa*I and has an end that can be inserted into a digestion site of *Eco*RI. The recognition sequence of *Bsa*I exists at such a position that the fragment is digested at an end of the partial sequence upon digestion with *Bsa*I after ligation. At one end of each of insert fragments that are not the insert fragment first inserted, a sequence that contains the recognition sequence of *Bsa*I and forms an end ligatable to an end formed by digestion with *Eco*RI is added to the partial sequence. The added sequence has such a length that digestion occurs at an end of the partial sequence upon digestion with *Esa*I after ligation. The other end of each of insert fragments that are not the insert fragment first inserted is made an end ligatable to an end formed by digestion with *Bsa*I. That is, the end of an insert fragment that is not the insert fragment inserted first has a shape that can be ligated to the end formed by digestion with *Bsa*I in the first vector. When the vector to which the insert fragment is inserted is digested with *Bsa*I, an end formed by digestion with *Bsa*I is formed at the end of the partial sequence. Therefore, an end of an insert fragment subsequently inserted has such a shape that a segment overlapping with the partial sequence of the insert fragment previously inserted serves as a protruding strand.

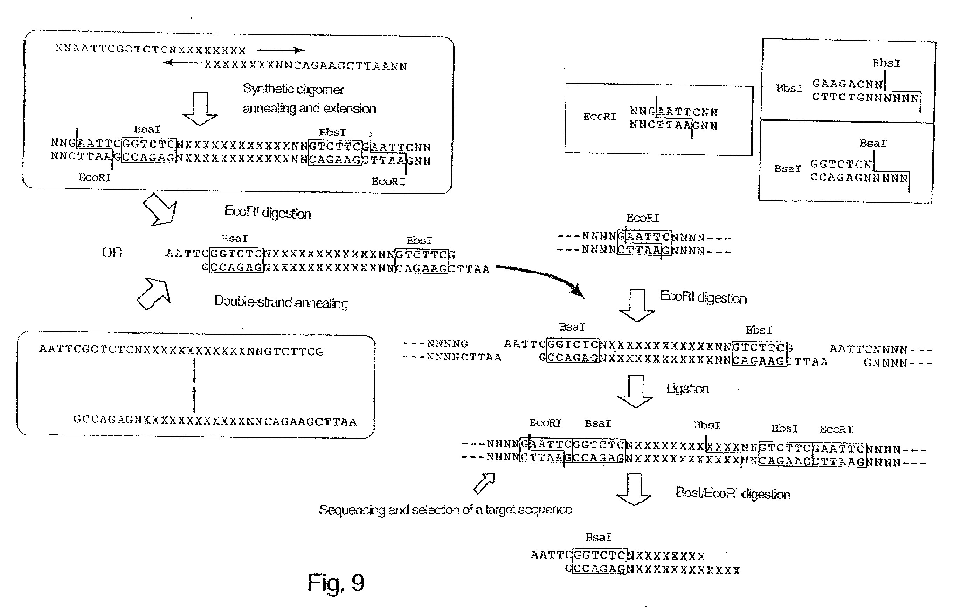

Such an insert fragment can be obtained by a method of synthesizing such two oligomers through chemical synthesis reactions that they form a target fragment upon annealing and then annealing these oligomers, a method of synthesizing, through chemical synthesis reactions, two oligomers designed so that a recognition sequence of a restriction enzyme exists on each 5' end side and that their 3' end sides anneal to each other, annealing these oligomers, then ' extending the annealed fragment by using a DNA polymerase and digesting the extended fragment with the restriction enzyme, or the like. Further, the insert fragment can also be obtained by the insert fragment-producing method of the present invention explained below (see Fig. 9).

When the target sequence has the recognition sites of *Bsa*I and *Eco*RI, other restriction enzymes are selected. Alternatively, the recognition sequences are changed when partial sequences are designed so that the ligated partial sequences do not have the recognition sites of *Bsa*I and *Eco*RI.

The insert fragment first inserted is prepared as a fragment that can be inserted into the digestion site of *Eco*RI and inserted into a vector containing *Eco*RI site. As a result, a vector can be obtained in which the recognition sequence of *Bsa*I exists between the digestion site of *Bsa*I and the digestion site of *Eco*RI.

The obtained vector is successively digested with *Bsa*I and *Eco*RI. This digestion provides two fragments. A longer fragment out of these fragments and one of the DNA fragments (one containing a partial sequence at an end) are ligated to obtain a vector.

Since the vector obtained by the ligation is a vector in which the recognition sequence of *Bsa*I exists between the digestion site of *Bsa*I and the digestion site of *Eco*RI, this vector is used in the same manner as the aforementioned vector, i.e., it is successively digested with *Bsa*I and *Eco*RI in the same manner as described above and ligated with a subsequent insert fragment (one containing a partial sequence adjacent to the partial sequence inserted immediately precedently) to obtain a vector. Until all the prepared insert fragments are ligated, successive digestion with *Bsa*I and *Eco*RI and ligation are repeated.

Thereafter, when a sequence is changed in designing of the partial sequences, the sequence is restored by a method such as site-directed mutagenesis.

Following the above procedure, DNA having the target sequence is produced.

### <3> Insert fragment-producing method of the present invention and vector used therefor

The present invention also provides a method for producing an insert fragment used for the present invention and a vector used therefor.

The insert fragment can be obtained by a usual method such as a method of synthesizing such two oligomers through chemical synthesis reactions that they form a target fragment upon annealing and then annealing these oligomers and a method of synthesizing, through chemical synthesis reactions, two oligomers designed so that a recognition sequence of restriction enzyme exists on each 5' end side and that their 3' end sides anneal to each other, annealing these oligomers, then extending the annealed fragment by using a DNA polymerase and digesting the extended fragment with the restriction enzyme. However, due to possibilities of errors in annealing, errors in uptake of nucleotides during the extension and inhibition of the reaction by impurities during the synthesis of the oligomers, it is not so likely that a fragment obtained by such a method should contain a target fragment. Therefore, after the insert fragments are ligated, sequencing must be performed to select a fragment incorporated with the target fragment. Both strands need to be read from both sides in order to reliably determine the sequence. In this case, however, the longer the synthesized strands become, the higher the cost becomes, since customized primers are required for the sequencing. Further, since this operation must be repeated for each incorporation, a very long period of time is required.

According to the insert fragment-producing method of the present invention, sequencing and production of an insert fragment can be easily performed by once incorporating a partial sequence into a vector in which recognition sequences of restriction enzymes are positioned in a specific manner. Consequently, if the target sequence is once determined, the factors to be considered in the design of the insert fragment are reduced. Therefore, the insert fragment can be easily designed, and selection of the fragments can be simultaneously performed after the incorporation into the vector.

An insert fragment-producing method according to a first embodiment of the present invention is a method for producing a DNA fragment used for the production method of the present invention, in which one end of the DNA fragment is formed by adding a sequence to a partial sequence, the added sequence forming an end ligatable to an end formed by digestion with the second restriction enzyme, to which the DNA fragment is ligated, and the added sequence having such a length that digestion occurs at an end of the partial sequence upon digestion with the first restriction enzyme after the ligation, and the other end is an end ligatable to an end formed by digestion with the first restriction enzyme, to which the DNA fragment is ligated, which method comprises steps of:
(a) preparing a vector having a recognition sequence of a third restriction enzyme which generates a digestion site having the same shape as that of a digestion site of the first restriction enzyme, and a recognition sequence of a fourth restriction enzyme, in which the recognition sequence of the fourth restriction enzyme exists between the recognition sequence and the digestion site of the third restriction enzyme,
(b) preparing a fragment comprising a partial sequence of which both ends are ligatable to an end formed by digestion with the fourth restriction enzyme,
(c) digesting the vector prepared in the step (a) with the fourth restriction enzyme and ligating the fragment obtained by the digestion with the fourth restriction enzyme and the DNA fragment prepared in the step (b),
(d) analyzing nuoleotide sequences of clones obtained by the ligation to select a clone having the target partial sequence, and
(e) successively digesting the selected clone with the third restriction enzyme and the fourth restriction enzyme,
   wherein
   sequences serving as the recognition sequences of the third restriction enzyme and the fourth restriction enzyme are provided in the vector and the fragment prepared in the steps (a) and (b), respectively, so that a predetermined DNA fragment is excised upon digestion of the vector obtained in the step (c) with the third restriction enzyme.

The first restriction enzyme, the second restriction enzyme and the insert fragment are as described in the explanations of the production method of the present invention.

The third restriction enzyme is a distant digestion-type enzyme that has a digestion site having the same shape as that of the digestion site of the first restriction enzyme. The third restriction enzyme may be the same as the first restriction enzyme.

The fourth restriction enzyme is not particularly limited so long as the recognition sequence and the digestion site thereof are specific. The same restriction enzyme as the second enzyme may be used.

The type of the vector is not particularly limited, and it may be a vector derived from a plasmid, phage, virus, artificial yeast chromosome or the like. Although the size of the vector is not particularly limited either, a smaller size of the segment other than an inserted sequence is preferred in view of efficient replication. The vector can be produced according to usual gene recombination techniques.

In the step (a), a vector having the recognition sequences of the third restriction enzyme and the fourth restriction enzyme is prepared, in which the recognition sequence of the fourth restriction enzyme exists between the recognition sequence and the digestion site of the third restriction enzyme. Such a vector can be produced according to usual gene recombination techniques.

In the step (b), a fragment containing a partial sequence of which both ends are ends ligatable to an end formed by digestion with the fourth restriction enzyme is prepared. Such a fragment can be obtained by a usual method such as a method of synthesizing two oligomers through chemical synthesis reactions so that they should form a target fragment upon annealing and then annealing these oligomers (double-strand annealing method) and a method of synthesizing, through chemical synthesis reactions, two oligomers designed so that a recognition sequence of a restriction enzyme exists on each 5' end side and that their 3' end sides anneal to each other, annealing these oligomers, then extending the annealed fragment by using a DNA polymerase, and digesting the extended fragment with the restriction enzyme (synthetic oligomer annealing and extension method).

In the step (c), the vector prepared in the step (a) is digested with the fourth restriction enzyme, and the fragment obtained by the digestion with the fourth restriction enzyme and the DNA fragment prepared in the step (b) are ligated. The digestion can be performed under conditions suitable for the restriction enzyme used. The fragments can be ligated in a conventional manner.

In the vector and the fragment prepared in the steps (a) and (b), respectively, sequences serving as the recognition sequences of the third restriction enzyme and fourth restriction enzyme are provided so that a predetermined insert fragment is excised upon digestion of the clones obtained in the step (c) with the third restriction enzyme.

In the step (d), the nucleotide sequences of the clones obtained by the ligation are analyzed to select a clone having the target partial sequence. The nucleotide sequences can be analyzed in a conventional manner.

In the step (e), the selected clone is successively digested with the third restriction enzyme and the fourth restriction enzyme. The digestion can be performed under conditions suitable for the restriction enzymes used.

In an embodiment of this insert fragment-producing method, the recognition sequence of the third restriction enzyme and the recognition sequence of the fourth restriction enzyme are identical, the vector prepared in the step (a) has another recognition sequence of the third restriction enzyme, digestion with the third restriction enzyme based on this recognition sequence generates an end having the same shape as that of an end formed by digestion with the fourth restriction enzyme, two recognition sequences of the third restriction enzyme exist on the both sides of the digestion site of the fourth restriction enzyme, and digestion is performed only with the third restriction enzyme in the step (e). In this embodiment, since only one kind of restriction enzyme is used in the step (e), the digestion operation becomes easy.

Hereafter, there will be explained a case where *Ace*III and *Eco*RI are selected as the third restriction enzyme and the fourth restriction enzyme, respectively, as an example, with reference to Fig. 5. In Fig. S, nucleotides of partial sequences obtained by dividing the target sequence are represented by X, and arbitrary nucleotides are represented by N.

First, a vector containing recognition sequences of AceIII and *Eco*RI is prepared in which the recognition sequence of *Eco*RI exists between the recognition sequence and the digestion site of *Ace*III. The nucleotide sequence of a segment containing the recognition sequences in the vector is shown as SEQ ID NO: 8.

A fragment containing a partial sequence having an end ligatable to an end formed by digestion with *Eco*RI for both ends is prepared. Such a fragment can be obtained by a usual method such as a method of synthesizing two oligomers through chemical synthesis reactions so that they form a target fragment upon annealing and then annealing these oligomers (double-strand annealing method) and a method of synthesizing, through chemical synthesis reactions, two oligomers designed so that a recognition sequence of a restriction enzyme exists on each 5' end side and that their 3' end sides anneal to each other, annealing these oligomers, then extending the annealed fragment by using a DNA polymerase, and digesting the extended fragment with the restriction enzyme (synthetic oligomer annealing and extension method).

The prepared vector is digested with *Eco*RI, and the fragment obtained by the digestion with *Eco*RI and the prepared DNA fragment are ligated.

In the prepared vector and fragment, the sequences of the recognition sequences of *Ace*III and *Eco*RI are provided so that a predetermined insert fragment is excised upon digestion of the clone obtained by Ligation with *Ace*III.

The nucleotide sequences of the clones obtained by the ligation are analyzed to select a clone having the target partial sequence.

The selected vector is successively digested with *Ace*III and *Eco*RI. If there is used, as the vector, a vector having another recognition sequence of *Ace*III (parenthesized sequence in Fig. 5) in which digestion, based on this recognition sequence with *Ace*III generates an end having the same shape as a site digested with *Eco*RI and two recognition sequences of *Ace*III exist on the both sides of the digestion site of *Eco*RI, it is sufficient to use only *Ace*III to excise an insert fragment. The nucleotide sequences of the segments containing the recognition sequences of the restriction enzymes in the segment digested upon the excision are shown in SEQ ID NOS: 9 and 10.

The present invention also provides a vector used in this embodiment of the insert fragment-producing method, in which the vector comprises the recognition sequences of the third restriction enzyme and the fourth restriction enzyme, and the recognition sequence of the fourth restriction enzyme exists between the recognition sequence and the digestion site of the third restriction enzyme, wherein the vector further comprises another recognition sequence of the third restriction enzyme, digestion based on this recognition sequence with the third restriction enzyme generates an end having the same shape as a site digested with the fourth restriction enzyme, and two recognition sequences of the third restriction enzyme exist on the both sides of the digestion site of the fourth restriction enzyme.

In another embodiment of the aforementioned insert fragment-producing method, the vector prepared in the step (a) has another recognition sequence of the third restriction enzyme, and two recognition sequences of the third restriction enzyme are symmetrically positioned as also for their directions with respect to the recognition sequence of the fourth restriction enzyme, and digestion is performed only with the third restriction enzyme in the step (f).

When a fragment is incorporated into this vector, the target fragment cannot be excised unless the fragment is incorporated in a specific direction. However, certain fragments may be incorporated only in a specific direction depending on their sequences. According to this embodiment, the aforementioned problem does not arise because the target fragment can be excised irrespective of the direction in which the fragment is incorporated.

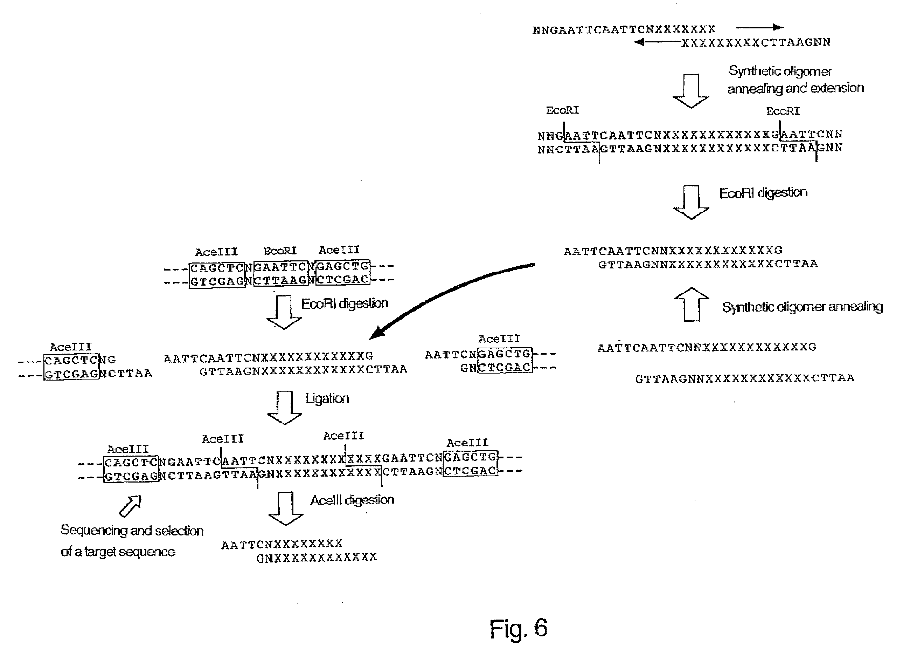

Hereafter, there will be explained a case where *Ace*III and *Eco*RI are selected as the third restriction enzyme and the fourth restriction enzyme, respectively, as an example, with reference to Fig. 6. In Fig. 6, nucleotides of partial sequences obtained by dividing the target sequence are represented by X, and arbitrary nucleotides are represented by N.

In this embodiment, as the vector, a vector is used in which another recognition sequence of *Ace*III is contained and two recognition sequences of *Ace*III are positioned symmetrically as also for their directions with respect to the recognition sequence of *Eco*RI. The nucleotide sequence of the segment containing the recognition sequence in the vector is shown as SEQ ID NO: 11.

In the prepared vector and fragment, sequences serving as the recognition sequences of *Ace*III and *Eco*RI are provided so that a predetermined insert fragment is excised upon digestion with *Ace*III of the clone obtained by ligation.

A clone in which the fragment is inserted into the digestion site of *Eco*RI in the vector and selected by sequencing is digested with *Ace*III. The nucleotide sequences of the segments containing the recognition sequences of the restriction enzymes in the segment to be digested upon the excision are shown as SEQ ID NOS: 12 and 13.

In this embodiment, two recognition sites of the fourth restriction enzyme may be used. In this case, operations may be the same as in the above embodiment except that two recognition sites are regarded as one site. In the embodiment using two recognition sites of the fourth restriction enzyme, it is preferred that the vector prepared in the step (a) has two recognition sequences of the fourth restriction enzyme, these two recognition sequences of the fourth restriction enzyme are positioned in directions inverse to each other, the recognition sequences of the third restriction enzyme are positioned symmetrically as also for their directions on both sides of two recognition sequences of the fourth restriction enzyme, and the fourth restriction enzyme forms a one nucleotide-protruding end at the 3' end. According to this embodiment, an end with which TA cloning can be performed is formed, and therefore loss can be further reduced by positioning the recognition sequences of the restriction enzymes on both sides thereof.

Examples of the restriction enzyme that forms a one nucleotide-protruding end at the 3' end include *Bmr*I, *Eam*1105I, *Bci*VI, *Mbo*II, *Mnl*I and so forth,

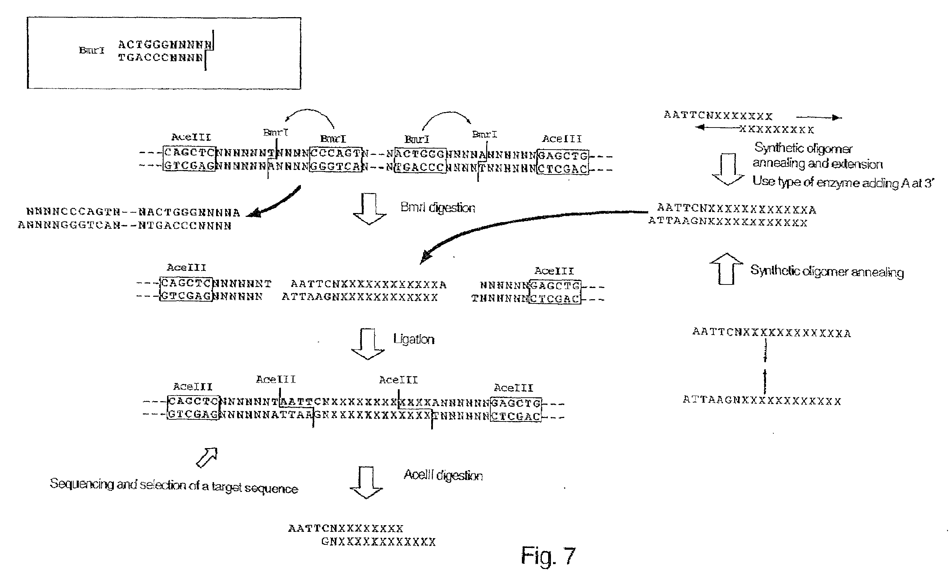

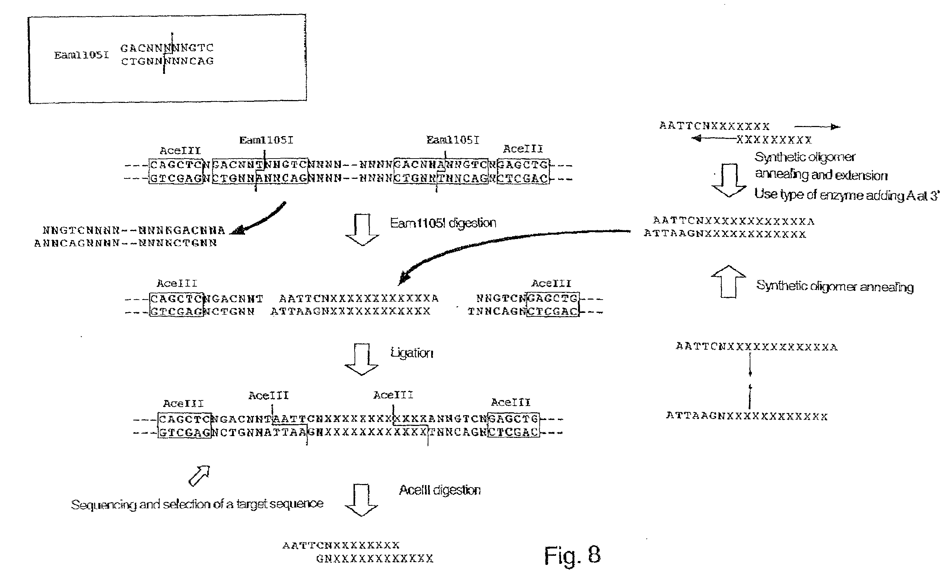

Hereafter, there will be explained a case where *Ace*III and *Bmr*I (Fig. 7) or *Eam*1105I (Fig. 8) are selected as the third restriction enzyme and the fourth restriction enzyme, respectively, as an example, with reference to Figs. 7 and 8. In Figs. 7 and 8, nucleotides of partial sequences obtained by dividing the target sequence are represented by X, and arbitrary nucleotides are represented by N.

In this embodiment, as the vector, a vector is used in which another recognition sequence of *Ace*III is contained and two recognition sequences of *Ace*III are positioned symmetrically as also for their directions with respect to two recognition sequences of *Bmr*I or *Eam*1105I. The nucleotide sequence of the segment containing the recognition sequences in the vector with *Bmr*I is shown in SEQ ID NO: 14. The nucleotide sequence of the segment containing the recognition sequences in the vector with *Eam*1105I is shown in SEQ ID NO: 17.

In the prepared vector and fragment, sequences serving as the recognition sequences of *Ace*III and *Bmr*I or *Eam*1105I are provided so that a predetermined insert fragment is excised upon digestion with *Ace*III of the vector obtained by ligation.

A vector in which the fragment is inserted into the digestion site of *Bmr*I or *Eam*1105I (between two digestion sites) and which is selected by sequencing is digested with *Ace*III. The nucleotide sequences of the segments containing the recognition sequences of the restriction enzymes in the segment to be digested upon the excision in the case of the vector with *Bmr*I are shown in SEQ ID NOS: 15 and 16. The nucleotide sequences of the segments containing the recognition sequences of the restriction enzymes in the segment to be digested upon the excision in the case of the vector with *Eam*1105I are shown in SEQ ID NOS: 18 and 19.

The present invention also provides a vector used in this embodiment of the insert fragment-producing method, in which the vector comprises the recognition sequences of the third restriction enzyme and the fourth restriction enzyme, and the recognition sequence of the fourth restriction enzyme exists between the recognition sequence and the digestion site of the third restriction enzyme, wherein the vector further comprises another recognition sequence of the third restriction enzyme, and two recognition sequences of the third restriction enzyme are positioned symmetrically as also for their directions with respect to the recognition sequence of the fourth restriction enzyme.

In this vector, there may be two recognition sites of the fourth restriction enzyme. Further, the vector may be a vector in which two recognition sequences of the fourth restriction enzyme are contained, these two recognition sequences of the fourth restriction enzyme are positioned in directions inverse to each other, the recognition sequences of the third restriction enzyme are positioned symmetrically as also for their directions on both sides of two recognition sequences of the fourth restriction enzyme, and the fourth restriction enzyme forms a one nucleotide-protruding end at the 3' end.

An insert fragment-producing method according to a second embodiment of the present invention is a method for producing a DNA fragment used in the production method of the present invention, in which one end of the DNA fragment is formed by adding a sequence to a partial sequence, the added sequence forming an end ligatable to an end formed by digestion with the second restriction enzyme, to which the DNA fragment is ligated, and the added sequence having such a length that digestion occurs at an end of the partial sequence upon digestion with the first restriction enzyme after the ligation, and the other end is an end ligatable to an end formed by digestion with the first restriction enzyme, to which the DNA fragment is ligated, which method comprises steps of:
(a) preparing a vector having a recognition sequence of the fourth restriction enzyme,
(b) preparing a fragment containing a partial sequence of which both ends are ligatable to an end formed by digestion with the fourth restriction enzyme, which has a recognition sequence of the third restriction enzyme so that digestion based on the recognition sequence of the third restriction enzyme generating a digested site having the same shape as that of a digested site based on the recognition sequence of the first restriction enzyme generates an end having the same shape as that of one end of the fragment, and has a recognition sequence of a fifth restriction enzyme generating an end having the same shape as an end generated by the third restriction enzyme so that digestion with the fifth restriction enzyme occurs at an end of the partial sequence,
(c) digesting the vector prepared in the step (a) with the fourth restriction enzyme and ligating the fragment obtained by the digestion with the fourth restriction enzyme and the DNA fragment prepared in the step (b),
(d) analyzing nucleotide sequences of clones obtained by the ligation to select a clone having the target partial sequence, and
(e) digesting the selected clone with the fourth restriction enzyme and the fifth restriction enzyme,
   wherein
   a sequence serving as the recognition sequence of the fifth restriction enzyme is provided in the vector and the fragment prepared in the steps (a) and (b), respectively, so that a predetermined DNA fragment is excised upon digestion of the clones obtained in the step (c) with the fifth restriction enzyme.

The fifth restriction enzyme is a distant digestion-type enzyme and has a digestion site having the same shape as that of the digestion site of the first restriction enzyme. The fifth restriction enzyme may be the same as the first restriction enzyme. The first to fourth restriction enzymes and the vector are the same as those in the first embodiment.

In the step (a), a vector having the recognition sequence of the fourth restriction enzyme is prepared. Such a vector can be produced according to usual gene recombination techniques.

In the step (b), there is prepared a fragment containing a partial sequence of which both ends are ligatable to an end formed by digestion with the fourth restriction enzyme, which has a recognition sequence of the third restriction enzyme so that digestion based on the recognition sequence of the third restriction enzyme generating a digested site having the same shape as that of a digested site based on the recognition sequence of the first restriction enzyme generates an end having the same shape as that of one end of the fragment, and has a recognition sequence of a fifth restriction enzyme generating an end having the same shape as an end generated by the third restriction enzyme so that digestion by the fifth restriction enzyme occurs at an end of the partial sequence. Such a fragment can be obtained by a usual method such as a method of synthesizing two oligomers through chemical synthesis reactions so that they form a target fragment upon annealing and then annealing these oligomers (double-strand annealing method) and a method of synthesizing, through chemical synthesis reactions, two oligomers designed so that a recognition sequence of a restriction enzyme exists on each 5' end side and that their 3' end sides anneal to each other, annealing these oligomers, then extending the annealed fragment by using a DNA polymerase, and digesting the extended fragment with the restriction enzyme (synthetic oligomer annealing and extension method).

In the step (c), the vector prepared in the step (a) is digested with the fourth restriction enzyme, and the fragment obtained by the digestion with the fourth restriction enzyme and the DNA fragment prepared in the step (b) are ligated. The digestion can be performed under conditions suitable for the restriction enzyme used. The fragments can he ligated in a conventional manner.

In the vector and fragment prepared in the steps (a) and (b), respectively, a sequence serving as the recognition sequence of the fifth restriction enzyme is provided so that a predetermined DNA fragment is excised upon digestion of the clones obtained in the step (c) with the fifth restriction enzyme.

In the step (d), the nucleotide sequences of the clones obtained by the ligation are analyzed to select a clone having the target partial sequence. The nucleotide sequences can be analyzed in a conventional manner.

In the step (e), the selected clone is digested with the fourth restriction enzyme and the fifth restriction enzyme. The digestion can be performed under conditions suitable for the restriction enzymes used. When the fragment digested with the fourth restriction enzyme cannot be digested with the fifth restriction enzyme, the fragment is successively digested with the fifth restriction enzyme and then the fourth restriction enzyme. When the fragment digested with the fourth restriction enzyme can be digested with the fifth restriction enzyme, the fragment may be digested with these enzymes simultaneously or successively. In the case of this successive digestion, the order may be arbitrary.

Hereafter, there will be explained a case where *Bsa*I, *Eco*RI and *Bbs*I are selected as the third restriction enzyme, the fourth restriction enzyme and fifth restriction enzyme, respectively, as an example, with reference to Fig. 9. In Fig. 9, nucleotides of partial sequences obtained by dividing the target sequence are represented by X, and arbitrary nucleotides are represented by N.

First, a vector having the recognition sequence of *Eco*RI is prepared.

There is prepared a fragment containing a partial sequence in which both ends are ligatable to an end formed by digestion with *Eco*RI, the recognition sequence of *Bsa*I is contained so that digestion based on the recognition sequence with *Bsa*I generates an end having the same shape as that of one end of this fragment and the recognition sequence of *Bbs*I is contained so that digestion with *Bbs*I causes digestion at an end of the partial sequence. Such a fragment can be obtained by a usual method such as a method of synthesizing two oligomers through chemical synthesis reactions so that they form a target fragment upon annealing and then annealing these oligomers (double-strand annealing method) and a method of synthesizing, through chemical synthesis reactions, two oligomers designed so that a recognition sequence of a restriction enzyme exists on each 5' end side and that their 3' end sides anneal to each other, annealing these oligomers, then extending the annealed fragment by using a DNA polymerase, and digesting the extended fragment with the restriction enzyme (synthetic oligomer annealing and extension method).

The prepared vector is digested with *Eco*RI, and the fragment obtained by the digestion with *Eco*RI and the prepared DNA fragment are ligated.

In the prepared vector and fragment, sequences serving as the recognition sequences of *Bbs*I and *Eco*RI are provided so that a predetermined insert fragment is excised upon digestion with *Bbs*I and *Eco*RI of the clone obtained by ligation.

The nucleotide sequences of the clones obtained by the ligation are analyzed to select a clone having the target partial sequence.

The selected clone is digested with *Bbs*I and *Eco*RI. The clone may be digested with these enzymes simultaneously or successively. In the case of successive digestion, the order may be arbitrary. The nucleotide sequences of the segments containing the recognition sequences of the restriction enzymes in the segment digested upon the excision are shown as SEQ ID NOS: 6 and 20.

## Claims

1. A vector comprising one recognition sequence of a first restriction enzyme of which digestion site exists at a particular position with respect to the recognition sequence and does not exist within the recognition sequence and one recognition sequence of a second restriction enzyme of which digestion site is specific, wherein a fragment obtained by digestion of the vector with the first restriction enzyme can be digested with the second restriction enzyme and a distance between the digestion site of the first restriction enzyme and the digestion site of the second restriction enzyme is 30 nucleotides or shorter.

2. A method for producing DNA having a target sequence, comprising steps of:
(1) preparing a plurality of double-stranded DNA fragments each having one of continuous partial sequences obtained by dividing the target sequence as a part thereof,
(2) preparing the vector as defined in Claim 1,
(3) digesting the vector with the first restriction enzyme,
(4) digesting the fragment obtained by the digestion with the first restriction enzyme with the second restriction enzyme,
(5) ligating a longer fragment out of the fragments obtained by the digestion with the second restriction enzyme and one of the DNA fragments prepared in the step (1) in a predetermined order, and
(6) repeating the steps of (3) to (5) by using a clone obtained by the ligation as the vector in the step (3) until all the DNA fragments prepared in the step (1) are ligated,
wherein
adjacent partial sequences overlap by nucleotides in a number of nucleotides in a protruding segment of a cohesive end to be generated at the digestion site of the first restriction enzyme,
one end of each DNA fragment prepared in the step (1) is formed by adding a sequence to each partial sequence, the added sequence forming an end ligatable to an end formed by digestion with the second restriction enzyme, to which the DNA fragment is ligated, and the added sequence having such a length that digestion occurs at an end of the partial sequence upon digestion with the first restriction enzyme after the ligation,
the other end of each DNA fragment is ligatable to an end formed by digestion with the first restriction enzyme, to which the DNA fragment is ligated, and
the first restriction enzyme and the second restriction enzyme are selected so that their recognition sequences do not exist within the ligated partial sequences.

3. The method according to Claim 2, which further comprises, when a sequence identical to the recognition sequence of the first restriction enzyme or the second restriction enzyme exists within the target sequence, a step of preparing the DNA fragments in the step (1) with changing the identical sequence to a different sequence and restoring the changed segment in the sequence of the DNA fragment obtained in the step (6) to the original sequence to produce DNA having the target sequence.

4. A method for producing DNA having a target sequence; comprising steps of:
(1) preparing a plurality of double-stranded DNA fragments each having one of continuous partial sequences obtained by dividing the target sequence as a part thereof,
(2) preparing the vector as defined in Claim 1, to which a DNA fragment being one of the DNA fragments prepared in the step (1) and having one of end partial sequences of the target sequence is inserted so that digestion occurs at an end of the partial sequence upon digestion with the first restriction enzyme,
(3) digesting the vector with the first restriction enzyme,
(4) digesting the fragment obtained by the digestion with the first restriction enzyme with the second restriction enzyme,
(5) lighting a longer fragment out of the fragments obtained by the digestion with the second restriction enzyme and one of the DNA fragments prepared in the step (1), and
(6) repeating the steps of (3) to (5) by using a clone obtained by the ligation as the vector in the step (3) until all the DNA fragments prepared in the step (1) are ligated,
wherein
adjacent partial sequences overlap by nucleotides in a number of nucleotides in a protruding segment of a cohesive end to be generated at the digestion site of the first restriction enzyme,
one end of each of the DNA fragments that are not the DNA fragment inserted in the step (2) is formed by adding a sequence to each partial sequence, the added sequence forming an end ligatable to an end formed by digestion with the second restriction enzyme, to which the DNA fragment is ligated, and the added sequence having such a length that digestion occurs at an end of the partial sequence upon digestion with the first restriction enzyme after the ligation,
the other end of each of the DNA fragments that are not the DNA fragment inserted in the step (2) is ligatable to an end formed by digestion with the first restriction enzyme, to which the DNA fragment is ligated, and
the first restriction enzyme and the second restriction enzyme are selected so that their recognition sequences do not exist within the ligated partial sequences.

5. The method according to Claim 4, which further comprises, when a sequence identical to the recognition sequence of the first restriction enzyme or the second restriction enzyme exists within the target sequence, a step of preparing the DNA fragments in the step (1) with changing the identical sequence to a different sequence and restoring the changed segment in the sequence of the DNA fragment obtained in the step (6) to the original sequence to produce DNA having the target sequence.

6. A method for producing a DNA fragment used in the production method as defined in any one of claims 2 to 5, in which one end of the DNA fragment is formed by adding a sequence to a partial sequence, the added sequence forming an end ligatable to an end formed by digestion with the second restriction enzyme, to which the DNA fragment is ligated, and the added sequence having such a length that digestion occurs at an end of the partial sequence upon digestion with the first restriction enzyme after the ligation, and the other end is an end ligatable to an end formed by digestion with the first restriction enzyme, to which the DNA fragment is ligated, which method comprises steps of:
(a) preparing a vector having a recognition sequence of a third restriction enzyme which generates a digestion site having the same shape as that of a digestion site of the first restriction enzyme, and a recognition sequence of a fourth restriction enzyme, in which the recognition sequence of the fourth restriction enzyme exists between the recognition sequence and the digestion site of the third restriction enzyme,
(b) preparing a fragment comprising a partial sequence of which both ends are ligatable to an end formed by digestion with the fourth restriction enzyme,
(c) digesting the vector prepared in the step (a) with the fourth restriction enzyme and ligating the fragment obtained by the digestion with the fourth restriction enzyme and the DNA fragment prepared in the step (b),
(d) analyzing nucleotide sequences of clones obtained by the ligation to select a clone having the target partial sequence, and
(e) successively digesting the selected clone with the third restriction enzyme and the fourth restriction enzyme,
wherein
sequences serving as the recognition sequences of the third restriction enzyme and the fourth restriction enzyme are provided in the vector and the fragment prepared in the steps (a) and (b), respectively, so that a predetermined DNA fragment is excised upon digestion of the clones obtained in the step (c) with the third restriction enzyme.

7. The method according to Claim 6, wherein the recognition sequence of the third restriction enzyme and the recognition sequence of the fourth restriction enzyme are identical,
the vector prepared in the step (a) has another recognition sequence of the third restriction enzyme, digestion with the third restriction enzyme based on this recognition sequence generates an end having the same shape as that of an end formed by digestion with the fourth restriction enzyme, two recognition sequences of the third restriction enzyme exist on the both sides of the digestion site of the fourth restriction enzyme, and
digestion is performed only with the third restriction enzyme in the step (e).

8. The method according to Claim 6, wherein the vector prepared in the step (a) has another recognition sequence of the third restriction enzyme, and two recognition sequences of the third restriction enzyme are symmetrically positioned as also for their directions with respect to the recognition sequence of the fourth restriction enzyme, and
digestion is performed only with the third restriction enzyme in the step (e).

9. The method according to Claim 8, wherein the vector prepared in the step (a) has two recognition sequences of the fourth restriction enzyme, two recognition sequences of the fourth restriction enzyme are positioned in directions inverse to each other, the recognition sequences of the third restriction enzyme are positioned symmetrically as also for their directions on both sides of two recognition sequences of the fourth restriction enzyme, and the fourth restriction enzyme forms a one nucleotide-protruding end at the 3' end.

10. A method for producing a DNA fragment used in the production method as defined in any one of Claims 2 to 5, in which one end of the DNA fragment is formed by adding a sequence to a partial sequence, the added sequence forming an end ligatable to an end formed by digestion with the second restriction enzyme, to which the DNA fragment is ligated, and the added sequence having such a length that digestion occurs at an end of the partial sequence upon digestion with the first restriction enzyme after the ligation, and the other end is an end ligatable to an end formed by digestion with the first restriction enzyme, to which the DNA fragment is ligated, which method comprises steps of:
(a) preparing a vector having a recognition sequence of the fourth restriction enzyme,
(b) preparing a fragment containing a partial sequence of which both ends are ligatable to an end formed by digestion with the fourth restriction enzyme, which has a recognition sequence of the third restriction enzyme so that digestion based or. the recognition sequence of the third restriction enzyme generating a digested site having the same shape as that of a digested site based on the recognition sequence of the first restriction enzyme generates an end having the same shape as that of one end of the fragment, and has a recognition sequence of a fifth restriction enzyme generating an end having the same shape as an end generated by the third restriction enzyme so that digestion with the fifth restriction enzyme occurs at an end of the partial sequence,
(c) digesting the vector prepared in the step (a) with the fourth restriction enzyme and ligating the fragment obtained by the digestion with the fourth restriction enzyme and the DNA fragment prepared in the step (b),
(d) analyzing nucleotide sequences of clones obtained by the ligation to select a clone having the target partial sequence, and
(e) digesting the selected clone with the fourth restriction enzyme and the fifth restriction enzyme,
wherein
a sequence serving as the recognition sequence of the fifth restriction enzyme is provided in the vector and the fragment prepared in the steps (a) and (b), respectively, so that a predetermined DNA fragment is excised upon digestion of the clones obtained in the step (c) with the fifth restriction enzyme.

11. A vector used for the method as defined in Claim 7, which comprises the recognition sequence of the third restriction enzyme, of which digestion site exists at a particular position with respect to the recognition sequence and does not exists within the recognition sequence, and the recognition sequence of the fourth restriction enzyme of which digestion site is specific, and in which the recognition sequence of the fourth restriction enzyme exists between the recognition sequence and the digestion site of the third restriction enzyme, wherein the vector has another recognition sequence of the third restriction enzyme, digestion based on this recognition sequence with the third restriction enzyme generates an end having the same shape as that obtained by digestion with the fourth restriction enzyme, and two recognition sequences of the third restriction enzyme exist on the both sides of the digestion site of the fourth restriction enzyme.

12. A vector used for the method as defined in Claim 8, which comprises the recognition sequence of the third restriction enzyme, of which digestion site exists at a particular position with respect to the recognition sequence and does not exists within the recognition sequence, and the recognition sequence of the fourth restriction enzyme of which digestion site is specific, and in which the recognition sequence of the fourth restriction enzyme exists between the recognition sequence and the digestion site of the third restriction enzyme, wherein the vector comprises another recognition sequence of the third restriction enzyme, and two recognition sequences of the third restriction enzyme are symmetrically positioned as also for their directions with respect to the recognition sequence of the fourth restriction enzyme.

13. The vector according to Claim 12, which comprises two recognition sequences of the fourth restriction enzyme, and in which two recognition sequences of the fourth restriction enzyme are positioned in directions inverse to each other, the recognition sequences of the third restriction enzyme are symmetrically positioned as also for their directions on both sides of two recognition sequences of the fourth restriction enzyme, and the fourth restriction enzyme forms a one nucleotide-protruding end at the 3' end.
